# EUROPEAN PATENT APPLICATION

(11) **EP 4 527 377 A1**
(43) Date of publication of application: **26.03.2025**
(21) Application number: 23806768.0
(22) Date of filing: 09.05.2023
(51) Int. Cl.: A61K 9/16, A61K 31/428, A61P 25/16

(54) **LONG-ACTING SUSTAINED-RELEASE PRAMIPEXOLE PREPARATION AND METHOD FOR PREPARING SAME**

(30) Priority: 16.05.2022 CN 202210528150
(71) Applicant: Sichuan Kelun Pharmaceutical Research Institute Co., Ltd., Chengdu, Sichuan 611138 (CN)
(72) Inventor: SU, Zhengxing, Chengdu, Sichuan 611138 (CN); DING, Duohao, Chengdu, Sichuan 611138 (CN); CHEN, Zhihao, Chengdu, Sichuan 611138 (CN); ZHAO, Dong, Chengdu, Sichuan 611138 (CN)
(74) Representative: Hoffmann Eitle
(86) International application number: PCT/CN2023/092984
(87) International publication number: WO 2023/221816

(57) **Abstract**

Provided are a long-acting sustained-release pramipexole preparation and a method for preparing same. The long-acting sustained-release pramipexole preparation comprises pramipexole pamoate or pramipexole palmitate, and a polylactic acid-glycolic acid copolymer or polylactic acid. The drug loading capacity of the preparation is 10%-50%. The molar ratio of lactic acid to glycolic acid of the polylactic acid-glycolic acid copolymer is 85:15-95:5. The microsphere is round in shape, smooth in surface and free of pores, and can effectively reduce the early burst release of the drug and solve the problem of high burst release of existing pramipexole pamoate microspheres. Also, the present invention features an effectively prolonged sustained-release period that solves the problem of the short release period of the existing pramipexole pamoate microspheres, and a smaller blood concentration fluctuation that leads to a more stable release.

## Description

### FIELD OF THE INVENTION

The present invention belongs to the field of pharmaceutical formulations, and specifically relates to a long-acting sustained-release pramipexole formulation and a method for preparing the same.

### BACKGROUND OF THE INVENTION

Relevant studies have shown that the incidence rate of Parkinson's disease (PD) in the population over 60 years old is 1.37%, and with the increasing aging of the Chinese population, the care and treatment of the large PD patient community is a social welfare issue that cannot be ignored.

Pramipexole is a second-generation potent, selective non-ergot dopamine D2 receptor agonist, used for the treatment of primary Parkinson's disease, and it can cover the entire stage of the disease treatment up to the advanced phase. It can significantly improve the motor symptoms of patients with early and advanced Parkinson's disease, as well as alleviate depressive symptoms associated with the disease. Pramipexole is the preferred medication recommended by Parkinson's disease treatment guidelines both domestically and internationally. However, the current market formulation of pramipexole hydrochloride tablets requires administration three times a day, which is a high frequency of dosing, Parkinson's patients often suffer from clinical symptoms such as memory decline, tremors in hands and feet, and difficulty swallowing, etc. Therefore, missed doses are common among patients, leading to deterioration of the condition, and even life-threatening choking and asphyxiation. A long-acting sustained-release formulation can avoid the first-pass effect, increase bioavailability, extend the dosing cycle and reduce the dosing frequency, greatly reducing the occurrence of poor compliance, bringing real clinical benefits to patients.

Biodegradable polymers polylactic acid-glycolic acid copolymer (PLGA) and polylactic acid (PLA) are certified by the United States Food and Drug Administration for their excellent biocompatibility and are included in the United States Pharmacopoeia as pharmaceutical excipients, making them suitable carriers for pramipexole sustained release. PLGA (or PLA) encapsulated long-acting sustained-release formulations, including microspheres, in-situ gels, *etc.,* can realize dosing from 1 week to 6 months. However, existing PLGA sustained-release formulations often face the challenges of high initial burst release and unstable drug release (large fluctuations in blood drug concentration), with peak-trough fluctuation (PTF) even exceeding oral formulations, which does not meet the basic requirements of formulation innovation for reduced toxicity and increased efficacy in China. Therefore, the development of pramipexole/PLGA or PLA sustained-release drugs with stable drug release and a long sustained-release period (one week or more) is of significant importance for the treatment of PD and has a huge prospect in clinical applications.

Pramipexole is a nucleophilic reagent with a certain degree of nucleophilicity, supplying electrons during the microsphere preparation process, acting as a Lewis base, causing degradation of the polymer material. Since this kind of degradation often occurs unconsciously and uncontrollably, the degradation of PLGA is irregular and uncontrollable. A regular overall decrease in PLGA molecular weight (weight-average molecular weight Mw, same hereinafter) will lead to a decrease in the viscosity of the oil phase, thereby affecting the final product's particle size, drug-loading capacity, and release cycle. In more cases, PLGA molecular weight does not decrease uniformly, specifically manifested as an increase in the molecular weight distribution (PDI), the generation of low polymers and oligomers, and an increase in monomer residues. This affects the batch-to-batch stability of the product (equivalent to poor process stability), the generation of process-related impurities (API reacting with monomers or oligomers). Moreover, due to the increase in low polymers, oligomers, and monomers which affects the compactness of the microsphere structure and the apparent porosity, the product's encapsulation rate and burst release situation are compromised.

Existing microsphere products have not controlled the degradation of polymer materials during preparation. Taking risperidone microspheres as an example: during preparation, a polymer material of higher molecular weight is selected, and microspheres are prepared when it degrades to the required molecular weight. This method cannot control the degradation of polymer materials; it can only prepare microspheres when degraded to the appropriate molecular weight. This method is neither economical nor reliable, and is a forced measure when the mechanism is not clear.

### SUMMARY OF THE INVENTION

The object of the present invention is to develop a pramipexole sustained-release medication with stable drug release and a long release cycle; another object is to control the compatibility of pramipexole with excipients to enhance product quality.

In a first aspect of the present invention, a long-acting sustained-release pramipexole formulation is provided, which comprises pramipexole pamoate or pramipexole palmitate, and polylactic acid-glycolic acid copolymer or polylactic acid, the drug loading capacity of the formulation is 10% to 55%, preferably 10% to 50%, more preferably 15% to 45%; and the molar ratio of lactic acid to glycolic acid of the polylactic acid-glycolic acid copolymer is 85:15 to 95:5.

In some embodiments of the first aspect of the present invention, the molecular weight of the polylactic acid-glycolic acid copolymer is from 10000 Da to 100000 Da; and the molecular weight of polylactic acid is from 10000 Da to 100000 Da.

In some embodiments of the first aspect of the present invention, the molar ratio of pamoic acid to pramipexole of the pramipexole pamoate is 1:2 or 1:1.

In some embodiments of the first aspect of the present invention, the molar ratio of palmitic acid to pramipexole of the pramipexole palmitate is 1:1.

In some embodiments of the first aspect of the present invention, the dosage form of the long-acting sustained-release pramipexole formulation is selected from microspheres, long-acting sustained-release particles, or subcutaneous implants.

In some embodiments of the first aspect of the present invention, the dosage form of the long-acting sustained-release pramipexole formulation is microspheres, with a drug loading capacity of 15-45%; preferably 30-45%, more preferably 35-40%.

In some embodiments of the first aspect of the present invention, the dosage form of the long-acting sustained-release pramipexole formulation is long-acting sustained-release particles or subcutaneous implants, with a drug loading capacity of 15-55%, preferably 30-55%, more preferably 40-55%.

A second aspect of the present invention provides a method for preparing pramipexole microspheres, comprising the following steps:
preparing an oil phase: dissolving pramipexole pamoate or pramipexole palmitate in a first solvent to obtain a first oil phase, dissolving polylactic acid-glycolic acid copolymer or polylactic acid in a second solvent to obtain a second oil phase, then mixing the first and second oil phases to obtain the oil phase;
preparing an aqueous solution of polyvinyl alcohol as the aqueous phase;
mixing the oil phase with the aqueous phase, emulsifying by high speed shear; solidifying the emulsified microspheres and volatilizing the solvent, washing with water, and freeze-drying.

In some embodiments of the second aspect of the present invention, the first solvent is selected from a group consisting of dimethyl sulfoxide, methanol, ethanol, isopropanol, tert-butanol, or N,N-dimethylformamide, preferably dimethyl sulfoxide or methanol; and the second solvent is selected from a group consisting of dichloromethane, chloroform, or ethyl acetate, preferably dichloromethane.

In some embodiments of the second aspect of the present invention, the weight ratio of pramipexole pamoate or pramipexole palmitate to polylactic acid-glycolic acid copolymer or polylactic acid is 1:1 to 1:5, preferably 1:1 to 1:3.

In some embodiments of the second aspect of the present invention, the weight to volume ratio (g/mL) of pramipexole pamoate or pramipexole palmitate to the first solvent is 1:1 to 1:10, preferably 1:3 to 1:8.

In some embodiments of the second aspect of the present invention, the weight to volume ratio (g/mL) of polylactic acid-glycolic acid copolymer or polylactic acid to the second solvent is 1:1 to 1:10, preferably 1:2 to 1:8.

In some embodiments of the second aspect of the present invention, the mixing of the first oil phase with the second oil phase is carried out under stirring at a temperature ≤25°C, preferably ≤ 10°C, more preferably ≤4°C, and the stirring time is ≤ 10h, preferably ≤2h, more preferably ≤ 1h, even more preferably ≤0.5h.

A third aspect of the present invention provides a method for preparing pramipexole subcutaneous implants or long-acting sustained-release particles, comprising the following steps: crushing pramipexole pamoate or pramipexole palmitate and polylactic acid-glycolic acid copolymer or polylactic acid and mixing, performing hot melt extrusion, followed by wire drawing of the extrudate to form short rods, thereby obtaining pramipexole subcutaneous implants; conducting wire drawing on the above-mentioned extrudate, pelletizing using a pelletizer, then crushing to the target particle size to obtain pramipexole sustained-release particles.

In some embodiments of the third aspect of the present invention, the weight ratio of pramipexole pamoate or pramipexole palmitate to polylactic acid-glycolic acid copolymer or polylactic acid is 5:1 to 1:1, preferably 3:1 to 1:1.

In some embodiments of the third aspect of the present invention, the extrusion temperature of the hot melt extrusion is 30-200°C, preferably 40-160°C, more preferably 80-120°C.

Compared with the prior art, the present invention achieves the following beneficial effects.

The microspheres of the present invention are well-rounded, with smooth surfaces and no pores, which can effectively reduce the initial burst release of the drug, solving the issue of high burst release seen in existing pramipexole pamoate microspheres. Meanwhile, it can effectively extend the sustained-release period, addressing the problem of the short release cycle of existing pramipexole pamoate microspheres, and results in smaller fluctuations in blood drug concentration, achieving a more stable release.

In the method for preparing the microspheres of the present invention, the raw and auxiliary materials are prepared separately and then mixed to obtain the oil phase. By controlling the mixing time of the raw and auxiliary materials, the mixing temperature of the oil phase, and selecting the type of excipients, the degradation rate of excipients during preparation is controlled. This control limits the generation of low polymers and oligomers during the degradation process of excipients, thereby improving the batch-to-batch stability of the product and reducing the generation of process-related impurities.

### BRIEF DESCRIPTION OF THE DRAWINGS

Figure 1 is a Scanning Electron Microscope (SEM) image of the pramipexole microspheres from Example 3.
Figure 2-1 and Figure 2-2 show the blood drug concentration curves in rats for the initial period (within 24 hours) and the entire release cycle, respectively, following a single dose of pramipexole microspheres from Example 3.
Figure 3-1 and Figure 3-2 show the blood drug concentration curves in rats for the initial period (within 24 hours) and the entire release cycle, respectively, following a single dose of pramipexole microspheres from Example 4.
Figure 4-1 and Figure 4-2 show the blood drug concentration curves in rats for the initial period (within 24 hours) and the entire release cycle, respectively, following a single dose of pramipexole microspheres from Comparative Example 1.
Figure 5-1 and Figure 5-2 show the blood drug concentration curves in rats for the initial period (within 24 hours) and the entire release cycle, respectively, following a single dose of pramipexole microspheres from Comparative Example 2.
Figure 6 shows the blood drug concentration curve in rats following a single dose of the pramipexole subcutaneous implant from Example 5.
Figure 7 shows the fitted blood drug concentration curve after multiple dosing of pramipexole microspheres from Example 3, using Phoenix WinNonlin software.
Figure 8 shows the fitted blood drug concentration curve after multiple dosing of pramipexole microspheres from Example 4, using Phoenix WinNonlin software.
Figure 9 shows the fitted blood drug concentration curve after multiple dosing of pramipexole microspheres from Comparative Example 1, using Phoenix WinNonlin software.
Figure 10 shows the fitted blood drug concentration curve after multiple dosing of pramipexole microspheres from Comparative Example 2, using Phoenix WinNonlin software.

### DETAILED DESCRIPTION OF THE INVENTION

The following specific embodiments are provided to further describe the above content of the present invention in detail. However, it should not be construed that the scope of the subject matter of the present invention is limited to the examples below. Any technical solution achieved based on the above content of the present invention falls within the scope of the present invention. Unless otherwise specified, the tests in the following examples can be conducted according to the methods described in the Pharmacopoeia of the People's Republic of China (2020 edition).

Unless explicitly stated otherwise, the molar ratio of pamoic acid to pramipexole is 1:2 in the "pramipexole pamoate" mentioned in the examples; the molar ratio of palmitic acid to pramipexole is 1:1 in the "pramipexole palmitate" mentioned in the examples.

Particle size detection: an appropriate amount of microsphere powder was dispersed in purified water, and the particle size of the microspheres was measured using a Master sizer 3000 laser particle size analyzer.

Drug loading capacity detection or calculation: firstly, the sustained-release formulation (such as microspheres, long-acting sustained-release particles, or subcutaneous implants) was destroyed with acetonitrile or dimethyl sulfoxide, so as to release pramipexole into the solution, then PLGA was precipitated with water, and the solution was centrifuged to determine the content of pramipexole in the supernatant. The drug loading capacity was calculated based on the weight ratio of the active component of the pramipexole salt to the sustained-release formulation (such as microspheres, long-acting sustained-release particles, or subcutaneous implants).

Calculation of encapsulation efficiency: Encapsulation efficiency = Drug loading capacity ÷ Theoretical drug loading capacity × 100%.

Calculation of solvent residue: Solvent residue = Weight of solvent ÷ Total weight of microspheres × 100%.

Viscosity determination: the well-dissolved oil phase was measured with a Brookfield DV2T rotational viscometer, using a No. 40 spindle, precisely taking 500 µl to determine the viscosity (cp) of the oil phase at 20°C and 4.0 rpm after being left to stand for different periods of time.

Molecular weight determination: gel permeation chromatography (GPC) was used with dichloromethane as the mobile phase and a flow rate of 1 mL/min through three Aglient PLgel (5 µm, 300 mm*7.5 mm) chromatography columns, with a sample size of 100 µl to detect the weight-average molecular weight (Mw) of PLGA or PLA.

As for the polymer materials involved in the present invention, for instance, PLGA 7525 3A is abbreviated as 7525 3A, where "7525" indicates the LA:GA block ratio is 75:25, "3" indicates the product intrinsic viscosity (IV) of about 0.3 dL/g, and "A" indicates carboxyl end-capping. PLA 2A represents PLA with an IV of about 0.2 dL/g and carboxyl end-capping.

### Example 1

2 g of pramipexole pamoate and 5 g of PLGA (7525 3A) were mixed evenly. At 25°C, they were dissolved in a mixed solvent of methanol and dichloromethane (15 mL, 25 mL), and the mixture was stirred for 0.5h until it was evenly mixed (recorded as oil phase sample ①).

2.2 g of pramipexole palmitate and 5 g of PLGA (7525 3A) were mixed evenly. At 25°C, they were dissolved in a mixed solvent of methanol and dichloromethane (15 mL, 25 mL), and the mixture was stirred for 0.5h until it was evenly mixed (recorded as oil phase sample ②).

2.2 g of pramipexole palmitate and 5 g of PLGA (7525 3A) were mixed evenly. At 10°C, they were dissolved in a mixed solvent of methanol and dichloromethane (15 mL, 25 mL), and the mixture was stirred for 0.5h until it was evenly mixed (recorded as oil phase sample ③).

2.2 g of pramipexole palmitate and 5 g of PLGA (7525 3A) were mixed evenly. At 4°C, they were dissolved in a mixed solvent of methanol and dichloromethane (15 mL, 25 mL), and the mixture was stirred for 0.5h until it was evenly mixed (recorded as oil phase sample ④).

2.2 g of pramipexole palmitate was dissolved in 15 mL of methanol to form the first oil phase; 5 g of PLGA (7525 3A) was dissolved in 25 mL of dichloromethane to form the second oil phase. At 25°C, the first and second oil phases were mixed evenly by stirring for 0.5h (recorded as oil phase sample ⑤).

2.2 g of pramipexole palmitate was dissolved in 15 mL of methanol to form the first oil phase; 5 g of PLGA (7525 3A) was dissolved in 25 mL of dichloromethane to form the second oil phase. At 25°C, the first and second oil phases were mixed evenly by stirring for 1h (recorded as oil phase sample ⑥).

2.2 g of pramipexole palmitate was dissolved in 15 mL of methanol to form the first oil phase; 5 g of PLGA (7525 3A) was dissolved in 25 mL of dichloromethane to form the second oil phase. At 25°C, the first and second oil phases were mixed evenly by stirring for 2h (recorded as oil phase sample ⑦).

2.2 g of pramipexole palmitate was dissolved in 15 mL of methanol to form the first oil phase; 5 g of PLGA (5050, 4.5A) was dissolved in 25 mL of dichloromethane to form the second oil phase. At 25°C, the first and second oil phases were mixed evenly by stirring for 0.5h (recorded as oil phase sample ⑧).

2.2 g of pramipexole palmitate was dissolved in 15 mL of methanol to form the first oil phase; 5 g of PLGA (8515, 4A) was dissolved in 25 mL of dichloromethane to form the second oil phase. At 25°C, the first and second oil phases were mixed evenly by stirring for 0.5h (recorded as oil phase sample ⑨).

2.2 g of pramipexole palmitate was dissolved in 15 mL of methanol to form the first oil phase; 5 g of PLA (2A) was dissolved in 25 mL of dichloromethane to form the second oil phase. At 25°C, the first and second oil phases were mixed evenly by stirring for 0.5h (recorded as oil phase sample ⑩).

The weight-average molecular weight (Mw) of PLGA or PLA in the above oil phase samples ①-⑩ was measured after different periods of standing. The results are shown in the table below.

| Periods of standing (h) | Molecular weight of PLGA or PLA (Mw/Da) | | | | | | | | | |
|---|---|---|---|---|---|---|---|---|---|---|
| | ① | ② | ③ | ④ | ⑤ | ⑥ | ⑦ | ⑧ | ⑨ | ⑩ |
| 0 | 37586 | 36604 | 3631 4 | 3607 9 | 37840 | 30228 | 3345 0 | 47138 | 4315 7 | 15588 |
| 0.5 | 35356 | 34160 | 3471 0 | 3569 4 | 37149 | 28145 | 2918 3 | 35762 | 4249 0 | 15541 |
| 4 | 35780 | 26093 | 2982 5 | 3361 8 | 33927 | 23538 | 2478 3 | 12137 | 4070 7 | 15282 |
| Differenc e after 4h | 1806 | 10511 | 6489 | 2461 | 3913 | 6690 | 8667 | 35001 | 2450 | 306 |

From the table above, it is understood that reducing the GA ratio in PLGA, lowering the mixing temperature of pramipexole with PLGA, and reducing the mixing time can all mitigate the reduction in PLGA's molecular weight in the oil phase. The preferred mixing temperature is ≤25°C, more preferably ≤10°C, and most preferably ≤4°C. Dissolving pramipexole and PLGA separately before mixing can reduce the mixing time of pramipexole with PLGA, which can also achieve the goal of inhibiting the degradation of PLGA during the oil phase preparation process.

### Example 2

To prepare the aqueous phase, 30 g of polyvinyl alcohol was dissolved in 3L of purified water and set aside for later use.

For the oil phase, 5 g of PLGA (5050 4.5A) was dissolved in a mixed solvent of methanol and dichloromethane (15 mL, 25 mL). At 25°C, the speed of the high-speed shear machine was adjusted to 1000rpm, and the oil phase was injected into the aqueous phase (1L) at a rate of 5 mL/min using a syringe for shear emulsification. After the emulsification was completed, the microsphere suspension was mechanically stirred at a rate of 300rpm to solidify for 4 hours. The microspheres were collected, washed with 1L of deionized water, and after freeze-drying, powder microspheres were obtained (recorded as microsphere sample ①).

2.2 g of pramipexole palmitate and 5 g of PLGA (5050 4.5A) were mixed and then dissolved in a mixed solvent of methanol and dichloromethane (15 mL, 25 mL), forming the oil phase. At 25°C, the speed of the high-speed shear machine was adjusted to 1000rpm, and the oil phase was injected into the aqueous phase (1L) at a rate of 5 mL/min using a syringe for shear emulsification. After the emulsification was completed, the microsphere suspension was mechanically stirred at a rate of 300rpm to solidify for 4 hours. The microspheres were collected, rinsed with 1L deionized water, and after freeze-drying, powder microspheres were obtained (recorded as microsphere sample ②).

2.2 g of pramipexole palmitate was dissolved in 15 mL of methanol as the first oil phase; 5 g of PLGA (5050 4.5A) was dissolved in 25 mL of dichloromethane as the second oil phase. At 4°C, the first and second oil phases were stirred for 0.5 hour to mix uniformly to form the oil phase. Under cold conditions at 4°C, the speed of the high-speed shear machine was adjusted to 1000rpm, and the oil phase was injected into the aqueous phase (1L) at a rate of 5 mL/min using a syringe for shear emulsification. After the emulsification was completed, the microsphere suspension was mechanically stirred at a rate of 300rpm to evaporate the solvent and solidify for 4 hours. The microspheres were collected, washed with 1L of deionized water, and after freeze-drying, powder microspheres were obtained (recorded as microsphere sample ③).

The test results of the particle size, drug loading capacity, encapsulation efficiency, and PLGA molecular weight of the above microsphere samples ①-③ are shown in the table below:

| Microsphere Sample | Particle Size/D₁₀, D₅₀, D₉₀/µm | Drug loading capacity /% | Encapsulation Efficiency/% | PLGA molecular weight/Da | PLGA reduction rate/% |
|---|---|---|---|---|---|
| ① | 13.09, 64.60, 148.48 | / | / | 47675 | / |
| ② | 7.01, 34.72, 90.76 | 10.61 | 60.66 | 32928 | 30.93% |
| ③ | 27.11, 87.12, 141.12 | 13.81 | 96.70 | 38441 | 19.37% |

From the table above, it is evident that microspheres prepared by the method of dissolving a mixture of pramipexole and PLGA in solvent to obtain the oil phase result in significant degradation of the polymer material. This leads to a decrease in the viscosity of the oil phase, a reduction in the particle size of the microspheres, and a very low encapsulation efficiency. However, microspheres prepared by the method of separately dissolving pramipexole and PLGA in solvent and then mixing to obtain the oil phase show only a slight reduction in the molecular weight of the polymer material, and the microspheres exhibit high drug loading capacity and encapsulation efficiency.

### Example 3

4.0 g of pramipexole pamoate (with a molar ratio of pamoate residue to pramipexole as 1:2) was dissolved in 17.5 mL of DMSO to form the first oil phase; 6 g of PLGA (8515, 5A) was dissolved in 17.5 mL of dichloromethane to form the second oil phase. The first and second oil phases were mixed and stirred at a temperature of 4°C for 0.5 hour to serve as the oil phase.

30 g of PVA was dissolved in 3L of water to form the aqueous phase.

Under shear conditions at a speed of 8000rpm, the oil and aqueous phases were mixed and shear emulsified. The emulsified microsphere solution was then stirred and solidified at a temperature of 4°C for 1 hour, the temperature was steadily increased to 25°C, and it was solidified for an additional 2 hours to evaporate the solvent from the solution.

Collection: the solidified microspheres were sieved to select the desired microspheres, which were then washed 2-3 times with purified water.

Freeze-drying: the microspheres were dried using a freeze-dryer or a three-in-one dryer to obtain the final product.

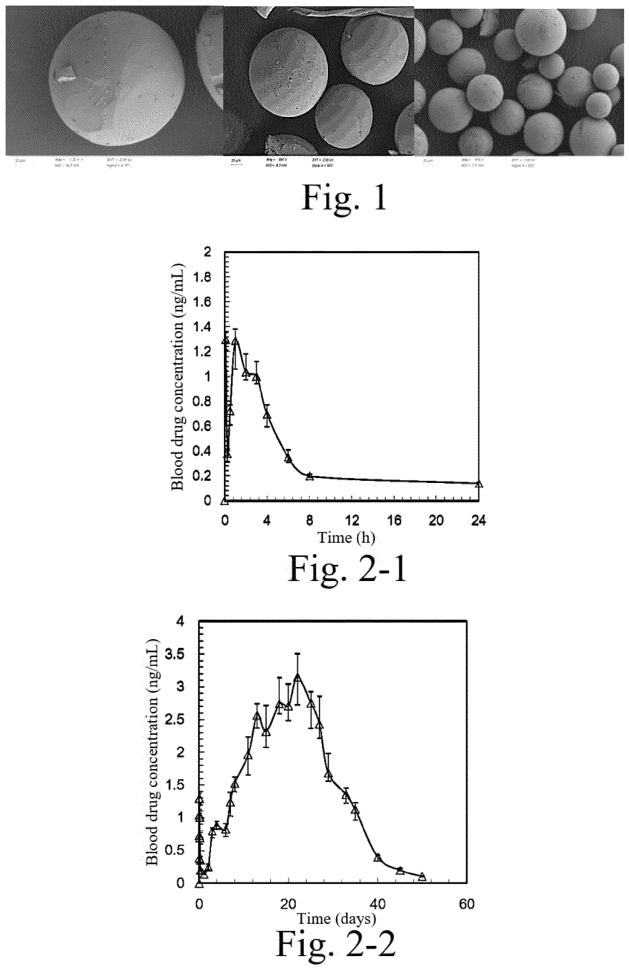

The prepared microspheres were examined with a scanning electron microscope, and the resulting images are as shown in Figure 1.

### Example 4

4.0 g of pramipexole pamoate (with a molar ratio of pamoate residue to pramipexole as 1:2) was dissolved in 17.5 mL of DMSO, forming the first oil phase; 6 g of PLA 2A was dissolved in 17.5 mL of dichloromethane, forming the second oil phase. The first and second oil phases were mixed and stirred at a temperature of 4°C for 0.5 hour to form the oil phase.

30 g of PVA was dissolved in 3L of water to form the aqueous phase.

Under shear conditions at a speed of 8000rpm, the oil and aqueous phases were mixed and shear emulsified. The emulsified microsphere solution was stirred and solidified at 4°C for 1 hour, then the temperature was steadily increased to 25°C, and it was solidified for an additional 2 hours to evaporate the solvent from the solution.

Collection: the solidified microspheres were sieved to select the desired microspheres, which were then washed 2-3 times with purified water.

Freeze-drying: the microspheres were dried using a freeze-dryer or a three-in-one dryer to obtain the final product.

### Example 5

40 g of pramipexole pamoate (with a molar ratio of pamoate residue to pramipexole as 1:2) and 25 g of PLA 5A were crushed, mixed evenly, and then subjected to hot melt extrusion. The extrudate was subjected to wire drawing to form short rods of 3×30 mm, which were then sterilized by irradiation at a condition of 8 kGy, thereby obtaining a pramipexole subcutaneous implant.

Hot melt extrusion parameters: a Thermo Fisher Pharma 11 and parallel counter-rotating twinscrew were used. The hot melt extruder was started with the following settings: feed temperature at room temperature, pushing temperature at 35~40°C, mixing temperature at 80~100°C, degassing temperature at 100°C, extrusion temperature at 80°C, and die temperature at 60°C. The pressure was set at 60 bar, extrusion speed at 100 rpm, and torque at 7~8 N·cm.

### Example 6

The extrudate from Example 5 was subjected to wire drawing and pelletized using a pelletizer, then crushed to particles with a particle size of 60-100 µm. The particles were then sterilized by irradiation under a condition of 8kGy to obtain long-acting sustained-release pramipexole particles.

### Comparative Example 1

4.0 g of pramipexole pamoate (with a molar ratio of pamoate residue to pramipexole as 1:2) was dissolved in 17.5 mL of DMSO to form the first oil phase; 6 g of PLGA (5050, 4.5A) was dissolved in 17.5 mL of dichloromethane to form the second oil phase. The first and second oil phases were mixed and stirred at a temperature of 4°C for 0.5 hour to form the oil phase.

30 g of PVA was dissolved in 3L of water to form the aqueous phase.

Under shear conditions at a speed of 8000rpm, the oil and aqueous phases were mixed and shear emulsified. The emulsified microsphere solution was then stirred and solidified at a temperature of 4°C for 1 hour, then the temperature was steadily increased to 25°C, and it was solidified for an additional 2 hours to evaporate the solvent from the solution.

Collection: the solidified microspheres were sieved to select the desired microspheres, which were then washed 2-3 times with purified water.

Freeze-drying: the microspheres were dried using a freeze dryer or a three-in-one dryer to obtain the final product.

### Comparative Example 2

4.0 g of pramipexole pamoate (with a molar ratio of pamoate residue to pramipexole as 1:2) was dissolved in 17.5 mL of DMSO to form the first oil phase; 6 g of PLGA (7525, 5A) was dissolved in 17.5 mL of dichloromethane to form the second oil phase. The first and second oil phases were mixed and stirred at a temperature of 4°C for 0.5 hour to form the oil phase.

30 g of PVA was dissolved in 3L of water to form the aqueous phase.

Under shear conditions at a speed of 8000rpm, the oil and aqueous phases were mixed and shear emulsified. The emulsified microsphere solution was then stirred and solidified at a temperature of 4°C for 1 hour, the temperature was then steadily increased to 25°C, and it was solidified for an additional 2 hours to evaporate the solvent from the solution.

Collection: the solidified microspheres were sieved to select the desired microspheres.

Freeze-drying: the microspheres were dried using a freeze dryer or a three-in-one dryer to obtain the final product.

### Experimental Example 1

The drug loading capacity, encapsulation efficiency, particle size, burst release, solvent residue, and molecular weight of the pramipexole microspheres from Examples 3-4 and Comparative Examples 1-2, the pramipexole subcutaneous implants from Example 5, and the long-acting sustained-release pramipexole particles from Example 6 were tested. The results are shown in the table below.

| Example No. | | Exampl e 3 | Example 4 | Example 5 | Example 6 | Compara tive Example 1 | Compara tive Example 2 |
|---|---|---|---|---|---|---|---|
| Drug loading capacity | | 37.28 % | 36.41 % | 54.21 % | 52.05% | 34.21% | 35.01 % |
| Encapsulation efficiency | | 93.2% | 91.0% | 88.1% | 84.6% | 85.5% | 87.5% |
| Partic le size | D50/µm | 82.74 | 81.82 | / | / | 81.02 | 85.43 |
| | Span | 1.65 | 1.55 | / | / | 1.61 | 1.96 |
| Burst release | | 0.88 % | 0.44 % | 2.89 % | 3.74 % | 7.74% | 3.52% |
| Solve nt residu e | Water | 0.65 % | 0.50 % | 0.44 % | 0.34 % | 0.65% | 0.74% |
| | DCM | 104 ppm | 73 ppm | / | / | 153ppm | 98 ppm |
| | DMSO | 0.76 % | 0.88 % | / | / | 0.56% | 0.54 % |
| PLA molecular weight (Da) | | / | / | 49900 | 47200 | / | / |

| | | | | | | | |
|---|---|---|---|---|---|---|---|
| Note 1: The release rate within 24 hours after administration to animals is defined as burst release, which is the proportion of the exposure amount in 24 hours to the total exposure amount over the entire period. The calculation formula is: Burst release = AUC₀₋₂₄ ÷ AUC₀₋ₗₐₛₜ * 100%. The samples from Examples 3-6 and Comparative Examples 1-2 were tested according to the method described in Experimental Example 2. | | | | | | | |

### Experimental Example 2

Samples from Examples 3-5 and Comparative Examples 1-2, along with a solid oral formulation (pramipexole hydrochloride tablet), were selected for animal tests. 25 male rats weighing around 250g were screened, randomly divided into groups (5 in each group), and given the sample mixed with the solvent (0.5% CMC-Na, 0.1% Tween-20, 0.9% NaCl) uniformly, at a drug dosage of 4 mg/kg by intramuscular injection, the administration was performed only once. Blood was collected intravenously before administration and after administration to determine the concentration of pramipexole in the plasma after administration.

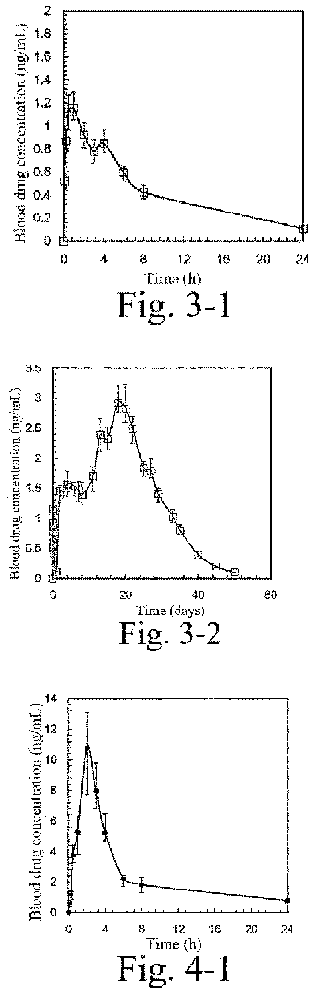

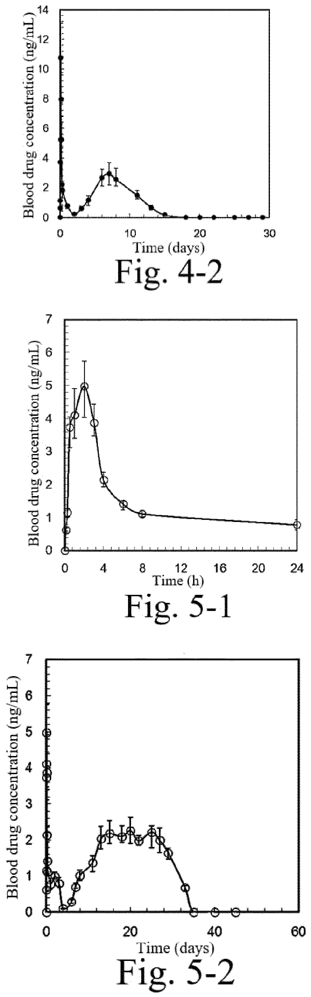

According to Figures 2-1, 3-1, 4-1, and 5-1, it is evident that the burst release of Examples 3-4 is significantly reduced compared to Comparative Examples 1-2. The present invention effectively reduces the early burst release of the drug by increasing the LA ratio in PLGA (LA:GA ≥ 85:15), solving the problem of high burst release in existing pramipexole pamoate microspheres.

Meanwhile, according to Figures 2-2, 3-2, 4-2, and 5-2, it is noticed that Examples 3-4 can effectively extend the sustained-release period of the formulation compared to Comparative Examples 1-2. This indicates that by increasing the LA ratio in PLGA (LA:GA ≥ 85:15), the present invention can effectively prolong the sustained-release period, resolving the issue of short release duration in existing pramipexole pamoate microspheres.

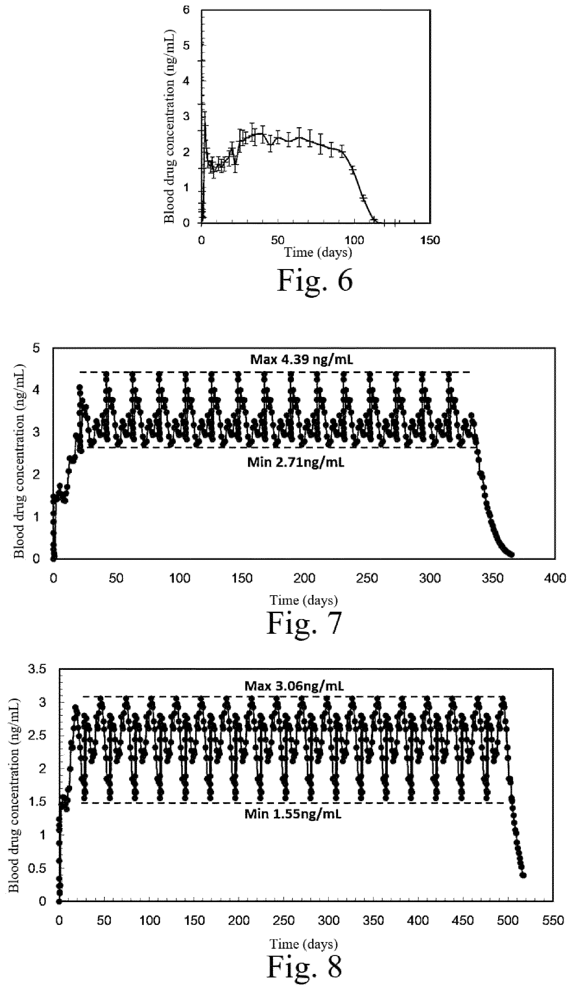

According to Figure 6, the pramipexole pamoate subcutaneous implants prepared in Example 5 can achieve administration every three months, with low burst release and stable drug release.

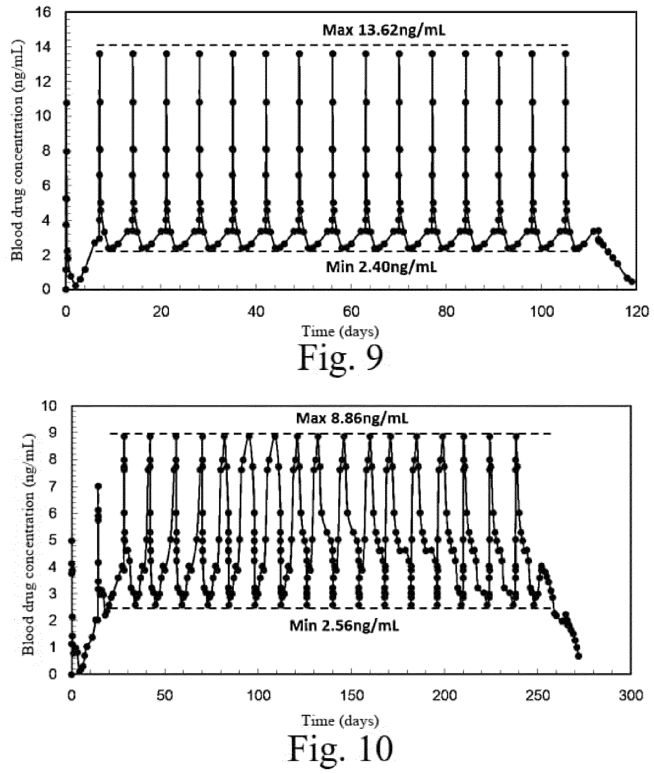

Based on the single-cycle blood drug concentration curves, Phoenix WinNonlin^{[2]} software was used to fit multiple dosages to evaluate the fluctuation of blood drug concentration after reaching the steady state in the body for the aforementioned Examples 3-4 and Comparative Examples 1-2. The results are shown in the table below and Figures 7-10.

Note[2]: Phoenix WinNonlin is the industry standard for analyzing pharmacokinetic and pharmacodynamic data, suitable for analysis platforms for non-clinical and clinical PK/PD studies from early non-clinical research to large clinical trials.

| | Cmin (ng/mL) | Cmax (ng/mL) |
|---|---|---|
| Pramipexole microspheres from Example 3 | 2.71 | 4.39 |
| Pramipexole microspheres from Example 4 | 1.55 | 3.06 |
| Pramipexole microspheres from Comparative Example 1 | 2.40 | 13.62 |
| Pramipexole microspheres from Comparative Example 2 | 2.56 | 8.86 |
| Solid oral formulation (pramipexole hydrochloride tablet) | 1.31 | 5.39 |

According to the above table and Figures 7-10, it can be concluded that the microspheres of the present invention provide a longer drug administration cycle, smaller fluctuations in blood drug concentration, and more stable release.

The technical features outlined in the preceding examples may be integrated in various configurations. To maintain conciseness, not all possible combinations of the various technical features in the preceding examples have been described. However, as long as the combinations of these technical features do not present conflicts, they should be considered within the scope disclosed by this specification.

The examples described above merely represent several embodiments of the invention, facilitating a concrete and detailed understanding of the technical solutions of the invention, but should not be construed as limiting the protection scope of the invention patent. It should be noted that, for those skilled in the art, several modifications and improvements can be made without departing from the concept of the invention, and these all fall within the protection scope of the invention. It should be understood that technical solutions obtained by those skilled in the art based on the technical solutions provided by the invention through logical analysis, reasoning, or limited experimentation are also within the scope of protection of the appended claims of the invention. Therefore, the scope of the patent protection for this invention should be defined by the contents of the appended claims, and the specification may be used to interpret the contents of the claims.

## Claims

1. **A long-acting** sustained-release pramipexole formulation, **characterized in that** it comprises pramipexole pamoate or pramipexole palmitate, and polylactic acid-glycolic acid copolymer or polylactic acid, wherein the drug loading capacity of the formulation is 10% to 55%, preferably 10% to 50%, more preferably 15% to 45%; and the molar ratio of lactic acid to glycolic acid of the polylactic acid-glycolic acid copolymer is 85:15 to 95:5.

2. The long-acting sustained-release pramipexole formulation according to claim 1, **characterized in that** the molecular weight of the polylactic acid-glycolic acid copolymer is from 10000 Da to 100000 Da; and the molecular weight of the polylactic acid is from 10000 Da to 100000 Da.

3. The long-acting sustained-release pramipexole formulation according to claim 1 or 2, **characterized in that** the molar ratio of pamoic acid to pramipexole of the pramipexole pamoate is 1:2 or 1:1.

4. The long-acting sustained-release pramipexole formulation according to any one of claims 1 to 3, **characterized in that** the dosage form of the long-acting sustained-release pramipexole formulation is selected from microspheres, long-acting sustained-release particles, or subcutaneous implants.

5. A method for preparing pramipexole microspheres, **characterized in that** it comprises the following steps:
preparing an oil phase: dissolving pramipexole pamoate or pramipexole palmitate in a first solvent to obtain a first oil phase, dissolving polylactic acid-glycolic acid copolymer or polylactic acid in a second solvent to obtain a second oil phase, then mixing the first and second oil phases to obtain the oil phase;
preparing an aqueous solution of polyvinyl alcohol as the aqueous phase;
mixing the oil phase with the aqueous phase, emulsifying by high speed shear; solidifying the emulsified microspheres and volatilizing the solvent, washing with water, and freeze-drying.

6. The method for preparing pramipexole microspheres according to claim 5, **characterized in that** the first solvent is selected from a group consisting of dimethyl sulfoxide, methanol, ethanol, isopropanol, tert-butanol, or N,N-dimethylformamide, preferably dimethyl sulfoxide or methanol; and the second solvent is selected from a group consisting of dichloromethane, chloroform, or ethyl acetate, preferably dichloromethane.

7. The method for preparing pramipexole microspheres according to claim 5 or 6, **characterized in that** the mixing of the first oil phase with the second oil phase is carried out under stirring at a temperature ≤ 25°C, preferably ≤ 10°C, more preferably ≤ 4°C, and the stirring time is ≤ 10h, preferably ≤ 2h, more preferably ≤ 1h, even more preferably ≤ 0.5h.

8. A method for preparing pramipexole subcutaneous implants or long-acting sustained-release particles, **characterized in that** it comprises the following steps: crushing pramipexole pamoate or pramipexole palmitate and polylactic acid-glycolic acid copolymer or polylactic acid and mixing, performing hot melt extrusion, followed by wire drawing of the extrudate to form short rods, thereby obtaining pramipexole subcutaneous implants; conducting wire drawing on the above-mentioned extrudate, pelletizing using a pelletizer, then crushing to the target particle size to obtain pramipexole sustained-release particles.

9. The method for preparing pramipexole subcutaneous implants or sustained-release particles according to claim 8, **characterized in that** the extrusion temperature for the hot melt extrusion is 30°C to 200°C, preferably 40°C to 160°C, and more preferably 80°C to 120°C.
